# EUROPEAN PATENT SPECIFICATION

(11) **EP 3 606 419 B1**
(45) Date of publication and mention of the grant of the patent: **27.12.2023**
(21) Application number: 18781037.9
(22) Date of filing: 29.03.2018
(51) Int. Cl.: A61B 5/332, A61B 5/304

(54) **CAPTURING ECG MEASUREMENTS IN A PORTABLE SENSOR DEVICE**
ERFASSUNG VON EKG-MESSUNGEN IN EINER TRAGBAREN SENSORVORRICHTUNG
CAPTURE DE MESURES D'ECG DANS UN DISPOSITIF DE CAPTEUR PORTABLE

(30) Priority: 04.04.2017 SE 1750413
(43) Date of publication of application: 12.02.2020
(73) Proprietor: Coala-Life AB, 753 19 Uppsala (SE)
(72) Inventor: SAMUELSSON, Magnus, 75319 Uppsala (SE)
(74) Representative: Kransell & Wennborg KB
(86) International application number: PCT/SE2018/000008
(87) International publication number: WO 2018/186780

(56) References cited:
- EP-A1- 0 784 996
- WO-A1-2015/113054
- CN-U- 205 625 919
- US-A1- 2005 182 335
- US-A1- 2007 293 774
- US-A1- 2011 251 817
- US-A1- 2011 288 605
- US-A1- 2012 157 867
- US-A1- 2013 085 367
- US-A1- 2013 281 816
- US-A1- 2013 338 529
- US-A1- 2013 345 540
- US-A1- 2013 345 540
- US-A1- 2015 005 609

## Description

### TECHNICAL FIELD

The invention relates to a method, a portable sensor device, a computer program and a computer program product for obtaining measurements for an Electrocardiogram (ECG).

### BACKGROUND

ECG is an established technology where electric signals generated by the body of a patient are measured and analysed. Traditionally, a number of electrodes are placed on the body at various places. A conductive gel is used to provide better conductive contact between the electrode and the skin. The patient typically lies down for minutes when the ECG is taken. The data detected using the electrodes is recorded and can be analysed by a professional, such as a physician or trained nurse. Once the measurement procedure is done, the conductive gel is wiped off.

While having proved useful, the traditional way of obtaining an ECG is not optimal in all cases. For instance, such an ECG needs to be measured in a clinic and the procedure is messy for the patient.

US 2013/338529 A1 discloses a bioelectric signal measurement apparatus that obtains an alternate current component and a direct current component of a bioelectric signal. The apparatus includes a plurality of biomedical electrodes, a switch unit, a differential amplification unit, a bioelectric signal extraction unit, and a timing control unit. The plurality of biomedical electrodes are brought into contact with a living body surface and disposed separately from each other. The switch unit short-circuits the biomedical electrodes via a predetermined short-circuit resistance. The differential amplification unit is connected to the biomedical electrodes and amplifies a difference of electric signals therefrom. The bioelectric signal extraction unit extracts a bioelectric signal from an output signal of the differential amplification unit from the release of short-circuit between the biomedical electrodes to the next timing of short-circuiting. The timing control unit controls timings for short-circuiting the biomedical electrodes and releasing the short-circuit therebetween, which are repeated.

Lately, portable sensor devices with integral electrodes for obtaining ECG data have been developed. These portable sensor devices allow users to capture ECG data at will and also without the use of conductive gel. This gives the user greater control over when to capture ECG data and also in a much more convenient and less messy way. However, when ECG data is captured using the portable sensor device, the ECG should be captured during a much shorter phase than traditionally. Moreover, the lack of conductive gel provides a more challenging situation for the electrodes to measure the electric signals from the body.

### SUMMARY

It is an object to provide a way to reduce measurement problems when capturing measurements for ECG using a portable sensor device.

According to a first aspect, it is presented a method for obtaining data for an Electrocardiogram, ECG, using a portable sensor device comprising two electrodes. The method comprising the steps of: receiving a trigger to obtain measurements for the ECG; setting at least one switch in a conducting state to close a connection between the two electrodes; setting the at least one switch in a blocking state to conductively separate the two electrodes; and capturing measurements for the ECG using the at least two electrodes.

The portable sensor device may comprise three electrodes, in which case the step of setting the at least one switch in a conducting state comprises closing a connection between the three electrodes and the step of setting the at least one switch in a blocking state comprises conductively separating the three electrodes.

The step of receiving a trigger may comprise receiving user input of a user interface element of the portable sensor device.

The step of receiving a trigger may comprise receiving a signal from an external device.

The step of capturing measurements may comprise comparing electrical signals from the two electrodes.

According to a second aspect, it is presented a portable sensor device for obtaining data for an Electrocardiogram, ECG. The portable sensor device comprises: two electrodes; at least one switch; a processor; and a memory storing instructions that, when executed by the processor, cause the portable sensor device to: receive a trigger to obtain measurements for the ECG; set the at least one switch in a conducting state to close a connection between the two electrodes; set the at least one switch in a blocking state to conductively separate the two electrodes; and capture measurements for the ECG using the at least two electrodes.

The portable sensor device may comprise three electrodes, in which case the instructions to set the at least one switch in a conducting state comprise instructions that, when executed by the processor, cause the portable sensor device to close a connection between the three electrodes and the instructions to set the at least one switch in a blocking state comprise instructions that, when executed by the processor, cause the portable sensor device to conductively separating the three electrodes.

The portable sensor device may further comprise a user interface element; in which case the instructions to receive a trigger comprise instructions that, when executed by the processor, cause the portable sensor device to receive user input of the user interface element.

The user interface element may be a push button.

The instructions to receive a trigger may comprise instructions that, when executed by the processor, cause the portable sensor device to receive a signal from an external device.

The instructions to capture measurements may comprise instructions that, when executed by the processor, cause the portable sensor device to compare electrical signals from the two electrodes.

The electrodes may be integral to the portable sensor device.

According to a third aspect, it is presented a computer program for obtaining data for an Electrocardiogram, ECG, using a portable sensor device comprising two electrodes. The computer program comprises computer program code which, when run on a portable sensor device causes the portable sensor device to: receive a trigger to obtain measurements for the ECG; set the at least one switch in a conducting state to close a connection between the two electrodes; set the at least one switch in a blocking state to conductively separate the two electrodes; and capture measurements for the ECG using the at least two electrodes.

According to a fourth aspect, it is presented a computer program product comprising a computer program according to the third aspect and a computer readable means on which the computer program is stored.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order disclosed, unless explicitly stated.

### BRIEF DESCRIPTION OF THE DRAWINGS

The invention is now described, by way of example, with reference to the accompanying drawings, in which:
Fig 1 is a schematic diagram illustrating an environment in which embodiments presented herein can be applied;
Fig 2 is a schematic diagram illustrating when the portable sensor device is used to capture measurements for ECG;
Figs 3A-3B are schematic diagrams of views illustrating a physical representation of the portable sensor device according to one embodiment;
Figs 4A-B are schematic graphs illustrating some issues which can occur in measurements obtained using portable sensor devices;
Fig 5 is a schematic diagram illustrating the portable sensor device of Fig 1 according to one embodiment with three electrodes;
Fig 6 is a schematic diagram illustrating the portable sensor device of Fig 1 according to one embodiment with two electrodes;
Fig 7 is a flow chart illustrating a method for obtaining data for an ECG using the portable sensor device of Fig 1 according to one embodiment; and
Fig 8 shows one example of a computer program product comprising computer readable means.

### DETAILED DESCRIPTION

The invention will now be described more fully hereinafter with reference to the accompanying drawings, in which certain embodiments of the invention are shown. This invention may, however, be embodied in many different forms and should not be construed as limited to the embodiments set forth herein; rather, these embodiments are provided by way of example so that this disclosure will be thorough and complete, and will fully convey the scope of the invention to those skilled in the art. Like numbers refer to like elements throughout the description.

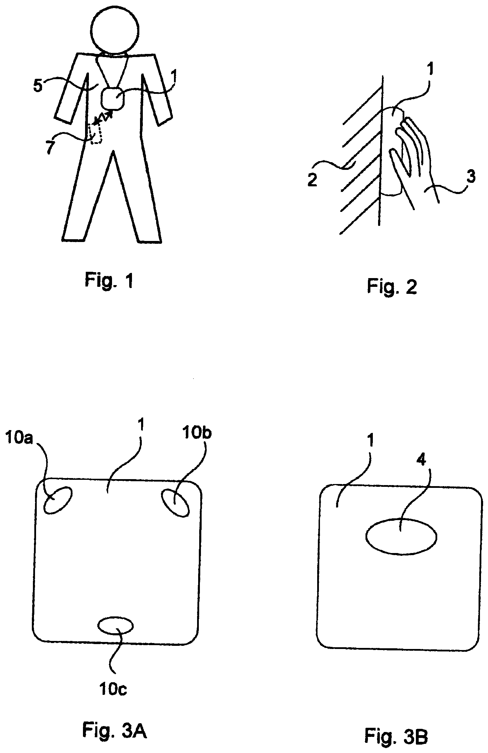

Fig 1 is a schematic diagram illustrating an environment in which embodiments presented herein can be applied.

It is here shown a user 5 carrying a portable sensor device 1 in a necklace strap. The user 5 also carries a smartphone 7 e.g. in a pocket. The portable sensor device 1 and the smartphone 7 can communicate over any suitable wireless interface, e.g. using Bluetooth or Bluetooth Low Energy (BLE), ZigBee, any of the IEEE 802.11x standards (also known as WiFi), etc.

Fig 2 is a schematic diagram illustrating when the portable sensor device 1 of Fig 1 is used to capture measurements for ECG. In order to capture measurements for ECG, the portable sensor device 1 is placed on the skin of the body 2 of the user. The user holds the portable sensor device 1 in place using a hand 3. It is to be noted that there are no loose electrodes for the measurement. Instead, the electrodes (as shown in Fig 3A and described below) are provided integral to the portable sensor device 1. Hence, the measurement for the ECG is captured simply by the user holding the portable sensor device 1 in contact with the skin of the body 2.

Figs 3A-3B are schematic diagrams of views illustrating a physical representation of the portable sensor device 1 of Fig 1 according to one embodiment.

In Fig 3A, a bottom view of the portable sensor device 1 is shown. There are a first electrode 10a, a second electrode 10b and a third electrode 10c. The electrodes 10a-c are placed on the casing of the portable sensor device 1 such that when the user places the portable sensor device 1 on the skin 2, all electrodes 10a-c are in contact with the skin 2. It is to be noted that the portable sensor device 1 could also be provided with two electrodes, four electrodes or any other suitable number of electrodes.

Optionally, the portable sensor device 1 can also comprise an electronic stethoscope (not shown).

In Fig 3B, a top view of the portable sensor device 1 is shown. Here, a user interface element 4 in form of a push button is shown. The push button can e.g. be used by the user to indicate when to start a measurement. It is to be noted that other user interface elements can be provided (not shown), e.g. more push buttons, Light Emitting Diodes (LEDs), a display, a speaker, a microphone, etc.

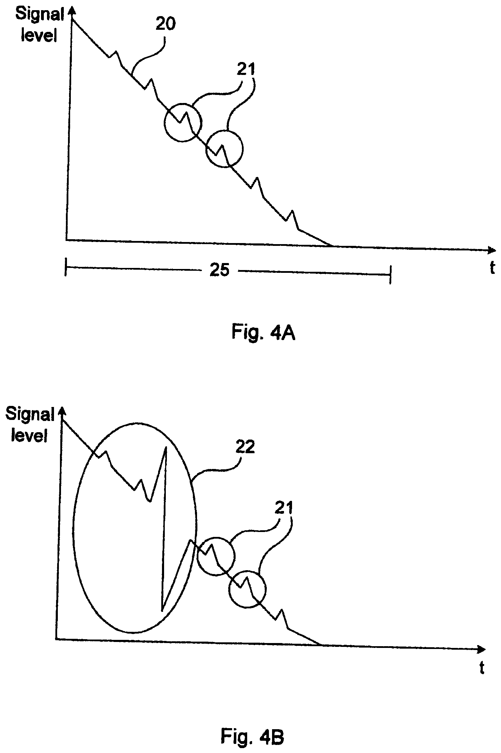

Figs 4A-B are schematic graphs illustrating some issues which can occur in measurements obtained using portable sensor devices. The horizontal axis represents time and the vertical axis represents signal level captured by a portable sensor device, e.g. as a voltage.

Looking first to Fig 4A, a signal 20 captured by a portable sensor device is shown. At time 0, the measurement starts. There are reoccurring sections 21 forming part of the signal 20. The reoccurring sections 21 are results of heartbeats of the user and those are the signals which are of interest for the ECG analysis. However, reoccurring sections 21 form part of the total signal 20 which also comprises a much larger bias which decreases over time. The bias is due to polarisation of the electrodes in the interface between the electrodes and the skin. The time 25 it takes for the bias to dissipate is significant. In some instances, this time has been measured to about 30 seconds.

Looking now to Fig 4B, another artefact 22 is shown. This artefact 22 is due to mechanical movement of the portable sensor device 1 in relation to the skin.

In conclusion, in comparison the wanted reoccurring signals 21, the bias and mechanical artefacts 22 make it difficult to extract the wanted signal.

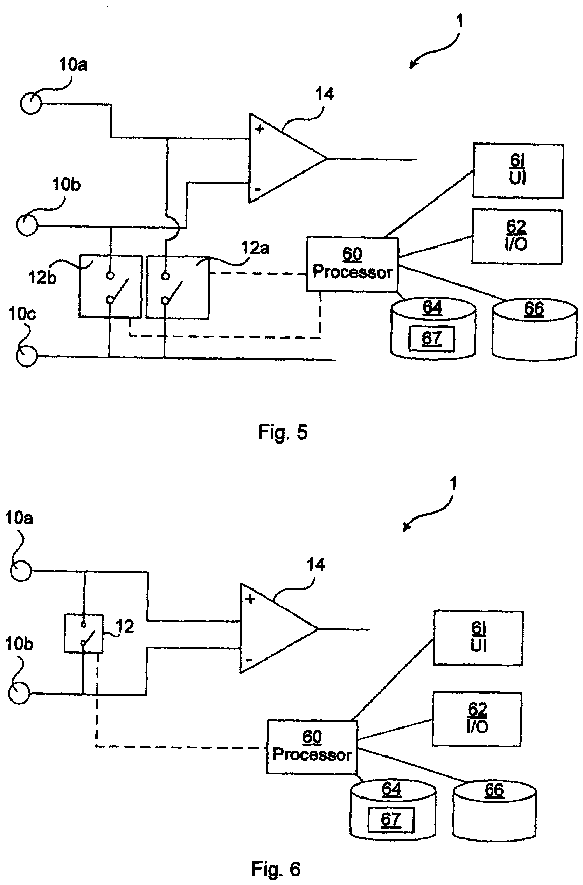

Fig 5 is a schematic diagram illustrating the portable sensor device 1 of Fig 1 according to one embodiment with three electrodes 10a-c. An output signal from the first electrode 10a is fed to a first input of an amplifier 14. An output signal from the second electrode 10b is fed to a second input of the amplifier 14. During a measurement phase, the amplifier 14 is then used to obtain an electric signal which is used as a measurement for the ECG. Moreover, there is a third electrode 10c, which can be used to filter out interference (e.g. from the mains grid) and to provide a suitable DC bias for the measurement.

The portable sensor device 1 also comprises a first switch 12a and a second switch 12b. The first switch 12a is provided between the first electrode 10a and the third electrode 10c. The second switch 12b is provided between the second electrode 10b and the third electrode 10c. A processor 60 controls the state of the switches 12a-b. The switches 12a-b are implemented using any suitable device which can be controlled to be in a conductive state or blocking state. For instance, the switches can be implemented using any suitable transistor or other semiconductor device.

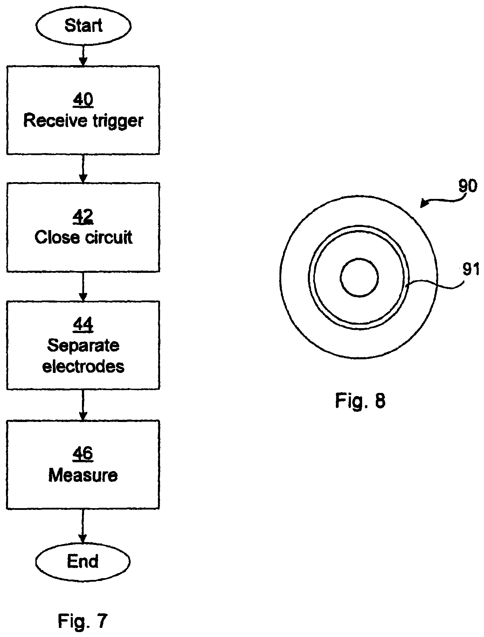

The processor 60 can be provided using any combination of one or more of a suitable central processing unit (CPU), multiprocessor, microcontroller, digital signal processor (DSP), application specific integrated circuit etc., capable of executing software instructions 67 stored in a memory 64, which can thus be a computer program product. The processor 60 can be configured to execute the method described with reference to Fig 7 below.

The memory 64 can be any combination of read and write memory (RAM) and read only memory (ROM). The memory 64 also comprises persistent storage, which, for example, can be any single one or combination of magnetic memory, optical memory, solid state memory or even remotely mounted memory.

A data memory 66 is also provided for reading and/or storing data during execution of software instructions in the processor 60. For instance, the data memory 66 can store digitized measurements. The data memory 66 can be any combination of read and write memory (RAM) and read only memory (ROM).

The portable sensor device 1 further comprises an I/O interface 62 for communicating with other external entities, such as the smartphone 7 of Fig 1. The I/O interface 62 can e.g. support any one or more of Bluetooth or Bluetooth Low Energy (BLE), ZigBee, any of the IEEE 802.11x standards (also known as WiFi), etc. The I/O interface 62 optionally also comprises a wired interface, e.g. for Universal Serial Bus (USB), FireWire, etc.

A user interface 4 is also provided, e.g. using a push button as shown in Fig 3B. Optionally, the user interface 4 comprises other user interface elements, e.g. more push buttons, Light Emitting Diodes (LEDs), a display, a speaker, a microphone, etc.

Other components of the portable sensor device 1, such as an analogue to digital (A/D) converter, are omitted in order not to obscure the concepts presented herein.

When a measurement is triggered to be performed, the control unit 60 sets both the first switch 12a and the second switch in a conducting state (i.e. closes the switches 12a-b). In this way, the voltage levels of all the three electrodes 10a-c are equalised. After the voltage levels are equalised, the switches 12a-b are set in a blocking state again, after which the measurements for the ECG can be captured.

It has been found that by equalising the voltages on the electrodes prior to measurement, the negative effects shown in Figs 4A-4B are significantly reduced. Hence, the wanted signal from the measured signal is easier to extract, leading to a better analysis of the state of the user based on the measurements of the ECG.

Fig 6 is a schematic diagram illustrating the portable sensor device 1 of Fig 1 according to one embodiment with two electrodes. The portable sensor device 1 is similar to that of Fig 5 and only differences to the embodiment of Fig 5 will be described.

Here, there is only one switch 12, which is provided between the first electrode 10a and the second electrode 10b. The switch is controlled in the same way as described above for the two switches 12a-b of Fig 5.

The embodiment of Fig 6 is a simpler and of a less costly construction than that of Fig 5, by having only two electrodes. However, this comes at a cost of not being able to provide the benefits of the third electrode explained above.

Fig 7 is a flow chart illustrating a method for obtaining data for an ECG using the portable sensor device of Fig 1 according to one embodiment.

In a *receive trigger* step 40, a trigger to obtain measurements for the ECG is received. The trigger can be in the form of user input of a user interface element of the portable sensor device, e.g. the pushbutton 4 of Fig 3B. Alternative, the trigger can be in the form of a signal received from an external device, such as the smartphone 7 of Fig 1.

In a *close connection* step 42, at least one switch is set in a conducting state to close a connection between the two electrodes. When the portable sensor device 1 comprises three electrodes, e.g. as shown in Fig 5, this step comprises closing a connection between all three electrodes. By closing the connection between the electrodes, the voltage of the electrodes all get equalised, i.e. they all obtain the same voltage level, which reduces the risk of the artefacts shown in Figs 4A-B and described above.

In a *separate electrodes* step 44, the at least one switch is set in a blocking state to conductively separate the two electrodes. When the portable sensor device 1 comprises three electrodes, e.g. as shown in Fig 5, this step comprises conductively separating all three electrodes. The electrodes need to be conductively separate for measurements to occur.

In a *measure* step 46, measurements are captured for the ECG using the at least two electrodes. The measurement can e.g. be captured by comparing electrical signals from the two electrodes, as shown in Figs 5 and 6 and described above. Once the measurements are recorded, these can be provided to an external device, such as the smartphone 7 of Fig 1. The measurements can be provided either progressively or in batch.

Fig 8 shows one example of a computer program product comprising computer readable means. On this computer readable means a computer program 91 can be stored, which computer program can cause a processor to execute a method according to embodiments described herein. In this example, the computer program product is an optical disc, such as a CD (compact disc) or a DVD (digital versatile disc) or a Blu-Ray disc. As explained above, the computer program product could also be embodied in a memory of a device, such as the computer program product 64 of Figs 5 and 6. While the computer program 91 is here schematically shown as a track on the depicted optical disk, the computer program can be stored in any way which is suitable for the computer program product, such as a removable solid state memory, e.g. a Universal Serial Bus (USB) drive.

The invention has mainly been described above with reference to a few embodiments. However, as is readily appreciated by a person skilled in the art, other embodiments than the ones disclosed above are equally possible within the scope of the invention, as defined by the appended patent claims.

## Claims

1. A method for obtaining data for an Electrocardiogram, ECG, using a portable sensor device (1) comprising a first electrode (10a), a second electrode (10b), a third electrode (10c), a first switch (12a) provided between the first electrode (10a) and the third electrode (10c), and a second switch (12b) provided between the second electrode (10b) and the third electrode (10c), the method comprising the steps of:
receiving (40) a trigger to obtain measurements for the ECG;
setting (42) the first switch (12a) in a conducting state to close a connection between the first electrode (10a) and the third electrode (10c), and setting the second switch (12b) in a conducting state to close a connection between the second electrode (10b) and the third electrode (10c);
setting (44) the first switch (12a) in a blocking state to conductively separate the first electrode (10a) and the third electrode (10c), and setting the second switch (12b) in a blocking state to conductively separate the second electrode (10b) and the third electrode (10c); and
capturing (46) measurements for the ECG using the three electrodes (10a, 10b, 10c).

2. The method according to claim 1, wherein the step of receiving (40) a trigger comprises receiving user input of a user interface element (4) of the portable sensor device (1).

3. The method according to claim 1, wherein the step of receiving (40) a trigger comprises receiving a signal from an external device (7).

4. The method according to any one of the preceding claims, wherein the step of capturing (46) measurements comprises comparing electrical signals from the two electrodes (10a, 10b).

5. A portable sensor device (1) for obtaining data for an Electrocardiogram, ECG, the portable sensor device (1) comprising:
a first electrode (10a), a second electrode (10b) and a third electrode (10c);
a first switch (12a) provided between the first electrode (10a) and the third electrode (10c), and a second switch (12b) provided between the second electrode (10b) and the third electrode (10c);
a processor (60); and
a memory (64) storing instructions (67) that, when executed by the processor, cause the portable sensor device to:
receive a trigger to obtain measurements for the ECG;
set the first switch (12a) in a conducting state to close a connection between the first electrode (10a) and the second electrode (10b);
set the second switch (12b) in a conducting state to close a connection between the second electrode (10b) and the third electrode (10c);
set the first switch (12a) in a blocking state to conductively separate the first electrode (10a) and the second electrode (10b);
set the second switch (12b) in a blocking state to conductively separate the second electrode (10b) and the third electrode (10c); and
capture measurements for the ECG using the three electrodes (10a, 10b, 10c).

6. The portable sensor device (1) according to claim 57, wherein the portable sensor device (1) further comprises a user interface element; and wherein the instructions to receive a trigger comprise instructions (67) that, when executed by the processor, cause the portable sensor device to receive user input of the user interface element (4).

7. The portable sensor device (1) according to claim 6, wherein the user interface element (4) is a push button.

8. The portable sensor device (1) according to any one of claims 5 to 7, wherein the instructions to receive a trigger comprise instructions (67) that, when executed by the processor, cause the portable sensor device to receive a signal from an external device (7).

9. The portable sensor device (1) according to any one of claims 5 to 8, wherein the instructions to capture measurements comprise instructions (67) that, when executed by the processor, cause the portable sensor device to compare electrical signals from the two electrodes (10a, 10b).

10. The portable sensor device (1) according to any one of claims 5 to 9, wherein the electrodes are integral to the portable sensor device (1).

11. A computer program (67, 91) for obtaining data for an Electrocardiogram, ECG, using a portable sensor device (1) comprising a first electrode (10a), a second electrode (10b), a third electrode (10c), a first switch (12a) provided between the first electrode (10a) and the third electrode (10c), and a second switch (12b) provided between the second electrode (10b) and the third electrode (10c), the computer program comprising computer program code which, when run on a portable sensor device (1) causes the portable sensor device (1) to:
receive a trigger to obtain measurements for the ECG;
set the first switch (12a) in a conducting state to close a connection between the first electrode (10a) and the second electrode (10b);
set the second switch (12b) in a conducting state to close a connection between the second electrode (10b) and the third electrode (10c);
set the first switch (12a) in a blocking state to conductively separate the first electrode (10a) and the second electrode (10b);
set the second switch (12b) in a blocking state to conductively separate the second electrode (10b) and the third electrode (10c); and
capture measurements for the ECG using the three electrodes (10a, 10b, 10c).

12. A computer program product (64, 90) comprising a computer program according to claim 11 and a computer readable means on which the computer program is stored.

## Patentansprüche

1. Verfahren zum Erhalten von Daten für ein Elektrokardiogramm (EKG) unter Verwendung einer tragbaren Sensorvorrichtung (1), die eine erste Elektrode (10a), eine zweite Elektrode (10b), eine dritte Elektrode (10c), einen ersten Schalter (12a), der zwischen der ersten Elektrode (10a) und der dritten Elektrode (10c) vorgesehen ist, und einen zweiten Schalter (12b), der zwischen der zweiten Elektrode (10b) und der dritten Elektrode (10c) vorgesehen ist, umfasst, wobei das Verfahren die folgenden Schritte umfasst:
Empfangen (40) eines Auslösers, um Messungen für das EKG zu erhalten;
Versetzen (42) des ersten Schalters (12a) in einen leitenden Zustand, um eine Verbindung zwischen der ersten Elektrode (10a) und der dritten Elektrode (10c) zu schließen, und
Versetzen des zweiten Schalters (12b) in einen leitenden Zustand, um eine Verbindung zwischen der zweiten Elektrode (10b) und der dritten Elektrode (10c) zu schließen;
Versetzen (44) des ersten Schalters (12a) in einen Sperrzustand, um die erste Elektrode (10a) und die dritte Elektrode (10c) leitend zu trennen, und
Versetzen des zweiten Schalters (12b) in einen Sperrzustand, um die zweite Elektrode (10b) und die dritte Elektrode (10c) leitend zu trennen; und
Erfassen von (46) Messungen für das EKG unter Verwendung der drei Elektroden (10a, 10b, 10c).

2. Verfahren nach Anspruch 1, wobei der Schritt des Empfangens (40) eines Auslösers das Empfangen einer Benutzereingabe von einem Benutzerschnittstellenelement (4) der tragbaren Sensorvorrichtung (1) umfasst.

3. Verfahren nach Anspruch 1, wobei der Schritt des Empfangens (40) eines Auslösers das Empfangen eines Signals von einer externen Vorrichtung (7) umfasst.

4. Verfahren nach einem der vorhergehenden Ansprüche, wobei der Schritt des Erfassens (46) von Messungen das Vergleichen elektrischer Signale von den beiden Elektroden (10a, 10b) umfasst.

5. Tragbare Sensorvorrichtung (1) zum Erhalten von Daten für ein Elektrokardiogramm (EKG), wobei die tragbare Sensorvorrichtung (1) Folgendes umfasst:
eine erste Elektrode (10a), eine zweite Elektrode (10b) und eine dritte Elektrode (10c);
einen ersten Schalter (12a), der zwischen der ersten Elektrode (10a) und der dritten Elektrode (10c) vorgesehen ist, und einen zweiten Schalter (12b), der zwischen der zweiten Elektrode (10b) und der dritten Elektrode (10c) vorgesehen ist;
einen Prozessor (60); und
einen Speicher (64), der Anweisungen (67) speichert, die, wenn sie vom Prozessor ausgeführt werden, die tragbare Sensorvorrichtung zu Folgendem veranlassen:
Empfangen eines Auslösers, um Messungen für das EKG zu erhalten;
Versetzen des ersten Schalters (12a) in einen leitenden Zustand, um eine Verbindung zwischen der ersten Elektrode (10a) und der zweiten Elektrode (10b) zu schließen;
Versetzen des zweiten Schalters (12b) in einen leitenden Zustand, um eine Verbindung zwischen der zweiten Elektrode (10b) und der dritten Elektrode (10c) zu schließen;
Versetzen des ersten Schalters (12a) in einen Sperrzustand, um die erste Elektrode (10a) und die zweite Elektrode (10b) leitend zu trennen;
Versetzen des zweiten Schalters (12b) in einen Sperrzustand, um die zweite Elektrode (10b) und die dritte Elektrode (10c) leitend zu trennen; und
Erfassen von Messungen für das EKG unter Verwendung der drei Elektroden (10a, 10b, 10c).

6. Tragbare Sensorvorrichtung (1) nach Anspruch 57, wobei die tragbare Sensorvorrichtung (1) ferner ein Benutzerschnittstellenelement umfasst; und
wobei die Anweisungen zum Empfangen eines Auslösers Anweisungen (67) umfassen, die, wenn sie von dem Prozessor ausgeführt werden, die tragbare Sensorvorrichtung veranlassen, Benutzereingaben des Benutzerschnittstellenelements (4) zu empfangen.

7. Tragbare Sensorvorrichtung (1) nach Anspruch 6, wobei das Benutzerschnittstellenelement (4) ein Druckknopf ist.

8. Tragbare Sensorvorrichtung (1) nach einem der Ansprüche 5 bis 7, wobei die Anweisungen zum Empfangen eines Auslösers Anweisungen (67) umfassen, die, wenn sie vom Prozessor ausgeführt werden, die tragbare Sensorvorrichtung veranlassen, ein Signal von einer externen Vorrichtung (7) zu empfangen.

9. Tragbare Sensorvorrichtung (1) nach einem der Ansprüche 5 bis 8, wobei die Anweisungen zum Erfassen von Messungen Anweisungen (67) umfassen, die, wenn sie vom Prozessor ausgeführt werden, die tragbare Sensorvorrichtung veranlassen, elektrische Signale von den beiden Elektroden (10a, 10b) zu vergleichen.

10. Tragbare Sensorvorrichtung (1) nach einem der Ansprüche 5 bis 9, wobei die Elektroden in die tragbare Sensorvorrichtung (1) integriert sind.

11. Computerprogramm (67, 91) zum Erhalten von Daten für ein Elektrokardiogramm (EKG) unter Verwendung einer tragbaren Sensorvorrichtung (1) mit einer ersten Elektrode (10a), einer zweiten Elektrode (10b), einer dritten Elektrode (10c), einem ersten Schalter (12a), der zwischen der ersten Elektrode (10a) und der dritten Elektrode (10c) vorgesehen ist, und einem zweiten Schalter (12b), der zwischen der zweiten Elektrode (10b) und der dritten Elektrode (10c) vorgesehen ist, wobei das Computerprogramm einen Computerprogrammcode umfasst, der, wenn er auf einer tragbaren Sensorvorrichtung (1) ausgeführt wird, die tragbare Sensorvorrichtung (1) zu Folgendem veranlasst:
Empfangen eines Auslösers, um Messungen für das EKG zu erhalten;
Versetzen des ersten Schalters (12a) in einen leitenden Zustand, um eine Verbindung zwischen der ersten Elektrode (10a) und der zweiten Elektrode (10b) zu schließen;
Versetzen des zweiten Schalters (12b) in einen leitenden Zustand, um eine Verbindung zwischen der zweiten Elektrode (10b) und der dritten Elektrode (10c) zu schließen;
Versetzen des ersten Schalters (12a) in einen Sperrzustand, um die erste Elektrode (10a) und die zweite Elektrode (10b) leitend zu trennen;
Versetzen des zweiten Schalters (12b) in einen Sperrzustand, um die zweite Elektrode (10b) und die dritte Elektrode (10c) leitend zu trennen; und
Erfassen von Messungen für das EKG unter Verwendung der drei Elektroden (10a, 10b, 10c).

12. Computerprogrammprodukt (64, 90), das ein Computerprogramm nach Anspruch 11 und ein computerlesbares Medium umfasst, auf dem das Computerprogramm gespeichert ist.

## Revendications

1. Procédé d'obtention de données pour un électrocardiogramme, ECG, en utilisant un dispositif capteur portable (1) comprenant une première électrode (10a), une deuxième électrode (10b), une troisième électrode (10c), un premier commutateur (12a) prévu entre la première électrode (10a) et la troisième électrode (10c), et un deuxième commutateur (12b) prévu entre la deuxième électrode (10b) et la troisième électrode (10c), le procédé comprenant les étapes de :
réception (40) d'un déclenchement pour obtenir des mesures pour l'ECG ;
mise (42) du premier commutateur (12a) dans un état conducteur pour fermer une connexion entre la première électrode (10a) et la troisième électrode (10c), et mise du deuxième commutateur (12b) dans un état conducteur pour fermer une connexion entre la deuxième électrode (10b) et la troisième électrode (10c) ;
mise (44) du premier commutateur (12a) dans un état de blocage pour séparer de manière conductive la première électrode (10a) et la troisième électrode (10c), et mise du deuxième commutateur (12b) dans un état de blocage pour séparer de manière conductive la deuxième électrode (10b) et la troisième électrode (10c) ; et
prise (46) de mesures pour l'ECG en utilisant les trois électrodes (10a, 10b, 10c).

2. Procédé selon la revendication 1, l'étape de réception (40) d'un déclenchement comprenant la réception d'une saisie utilisateur d'un élément interface utilisateur (4) du dispositif capteur portable (1).

3. Procédé selon la revendication 1, l'étape de réception (40) d'un déclenchement comprenant la réception d'un signal en provenance d'un dispositif externe (7).

4. Procédé selon l'une quelconque des revendications précédentes, l'étape de prise (46) de mesures comprenant la comparaison de signaux électriques provenant des deux électrodes (10a, 10b).

5. Dispositif capteur portable (1) pour obtenir des données pour un électrocardiogramme, ECG, le dispositif capteur portable (1) comprenant :
une première électrode (10a), une deuxième électrode (10b) et une troisième électrode (10c) ;
un premier commutateur (12a) prévu entre la première électrode (10a) et la troisième électrode (10e), et un deuxième commutateur (12b) prévu entre la deuxième électrode (10b) et la troisième électrode (10c) ;
un processeur (60) ; et
une mémoire (64) stockant des instructions (67) qui, lorsqu'elles sont exécutées par le processeur, amènent le dispositif capteur portable à :
recevoir un déclenchement pour obtenir des mesures pour l'ECG ;
mettre le premier commutateur (12a) dans un état conducteur pour fermer une connexion entre la première électrode (10a) et la deuxième électrode (10b) ;
mettre le deuxième commutateur (12b) dans un état conducteur pour fermer une connexion entre la deuxième électrode (10b) et la troisième électrode (10c) ;
mettre le premier commutateur (12a) dans un état de blocage pour séparer de manière conductive la première électrode (10a) et la deuxième électrode (10B) ;
mettre le deuxième commutateur (12b) dans un état de blocage pour séparer de manière conductive la deuxième électrode (10b) et la troisième électrode (10c) ; et prendre des mesures pour l'ECG en utilisant les trois électrodes (10a, 10b, 10c).

6. Dispositif capteur portable (1) selon la revendication 5, le dispositif capteur portable (1) comprenant en outre un élément interface utilisateur ;
et
les instructions pour recevoir un déclenchement comprenant des instructions (67) qui, lorsqu'elles sont exécutées par le processeur, amènent le dispositif capteur portable à recevoir une saisie utilisateur de l'élément interface utilisateur (4).

7. Dispositif capteur portable (1) selon la revendication 6, l'élément interface utilisateur (4) étant un bouton poussoir.

8. Dispositif capteur portable (1) selon l'une quelconque des revendications 5 à 7, les instructions pour recevoir un déclenchement comprenant des instructions (67) qui, lorsqu'elles sont exécutées par le processeur, amènent le dispositif capteur portable à recevoir un signal en provenance d'un dispositif externe (7) .

9. Dispositif capteur portable (1) selon l'une quelconque des revendications 5 à 8, les instructions de prise de mesures comprenant des instructions (67) qui, lorsqu'elles sont exécutées par le processeur, amènent le dispositif capteur portable à comparer des signaux électriques en provenance des deux électrodes (10a, 10b).

10. Dispositif capteur portable (1) selon l'une quelconque des revendications 5 à 9, les électrodes faisant partie intégrante du dispositif capteur portable (1) .

11. Programme informatique (67, 91) d'obtention de données pour un électrocardiogramme, ECG, en utilisant un dispositif capteur portable (1) comprenant une première électrode (10a), une deuxième électrode (10b), une troisième électrode (10c), un premier commutateur (12a) prévu entre la première électrode (10a) et la troisième électrode (10c), et un deuxième commutateur (12b) prévu entre la deuxième électrode (10b) et la troisième électrode (10c), le programme informatique comprenant du code de programme informatique qui, lorsqu'il tourne sur un dispositif capteur portable (1) amène le dispositif capteur portable (1) à :
recevoir un déclenchement pour obtenir des mesures pour l'ECG ;
mettre le premier commutateur (12a) dans un état conducteur pour fermer une connexion entre la première électrode (10a) et la deuxième électrode (10b) ;
mettre le deuxième commutateur (12b) dans un état conducteur pour fermer une connexion entre la deuxième électrode (10b) et la troisième électrode (10c) ;
mettre le premier commutateur (12a) dans un état de blocage pour séparer de manière conductive la première électrode (10a) et la deuxième électrode (10b) ;
mettre le deuxième commutateur (12b) dans un état de blocage pour séparer de manière conductive la deuxième électrode (10b) et la troisième électrode (10c) ; et
prendre des mesures pour l'ECG en utilisant les trois électrodes (10a, 10b, 10c).

12. Produit programme informatique (64, 90) comprenant un programme informatique selon la revendication 11 et un moyen lisible par ordinateur sur lequel le programme informatique est stocké.
